Europäisches Patentamt

European Patent Office

Office européen des brevets

(11) Publication number: **0 351 246**
**A2**

## EUROPEAN PATENT APPLICATION

(21) Application number: **89307194.4**

(22) Date of filing: **14.07.89**

(51) Int. Cl.⁵: **C 12 N 9/64**
**C 12 N 15/58, C 12 N 5/10,**
**A 61 K 37/547**

(30) Priority: **15.07.88 DK 3952/88**

(43) Date of publication of application:
**17.01.90 Bulletin 90/03**

(84) Designated Contracting States:
**AT BE CH DE ES FR GB GR IT LI LU NL SE**

(71) Applicant: **NOVO-NORDISK A/S**
**Novo Allé**
**DK-2880 Bagsvaerd (DK)**

(72) Inventor: **Petersen, Lars Christian**
**Havevej 4,**
**DK-2970 Hoersholm (DK)**

**Boel, Esper**
**Lyngbakkevej 25**
**DK-2840 Holte (DK)**

(74) Representative: **Brown, John David et al**
**FORRESTER & BOEHMERT Widenmayerstrasse 4/I**
**D-8000 München 22 (DE)**

(54) A tissue plasminogen activator analogue.

(57) A tissue plasminogen activator (t-PA) analogue in substantially inactivated one-chain form capable of being converted to the active two-chain form is modified in the region from position 414 to position 433. In particular, the t-PA analogue is one in which a positively charged amino acid residue in position 419 and optionally also in position 420 has been replaced by a negatively charged or uncharged amino acid residue to inhibit interaction with the amino acid residue in position 480.

**Description**

## A TISSUE PLASMINOGEN ACTIVATOR ANALOGUE

FIELD OF INVENTION

The present invention relates to a novel tissue plasminogen activator (t-PA) analogue, a DNA construct encoding the analogue, a pharmaceutical composition containing the analogue and a method of preparing the analogue.

BACKGROUND OF THE INVENTION

Delicate enzymatic mechanisms control the fluidity of blood. In response to injury, clotting factors ensure rapid coagulation of blood by conversion of plasma fibrinogen to insoluble fibrin. Fibrinolytic enzymes are responsible for the orderly removal of already formed fibrin clots. If these mechanisms become deranged clots may form inappropriately inside the vascular bed. This can impair the local flow of blood and result in tissue malfunction, followed later by necrosis and replacement with scar tissue. Depending on the site of damage the injured individual will suffer from one of a variety of diseases, including myocardial infarction, deep vein thrombosis, pulmonary embolism, and stroke, all of which are serious public health problems.

Tissue damage does not occur instantaneously upon obstruction of the blood flow, but rather develops over some hours. Therefore, rapid restoration of the blood supply to the endangered area will minimize permanent damage. It is commonly recognized that therapies ensuring the reopening of occluded vessels within a few hours would be of great benefit and have the potential of appreciably reducing the number of permanently disabled persons.

One approach to the problem has been to stimulate forma tion of natural fibrinolytic enzymes. The major fibrinolytic enzyme of the body is plasmin. It circulates in blood in the form of the proenzyme plasminogen, from which it is formed by proteolytic cleavage of a single peptide bond between Arg(561) and Val(562). This cleavage is catalyzed by the action of plasminogen activators, e.g. tissue plasminogen activator (t-PA). Plasminogen binds to fibrin clots, and degrades fibrin to soluble fragments when converted to plasmin.

However, plasmin is a nonspecific protease, and if it is formed in circulating blood it will degrade a variety of plasma proteins, including fibrinogen, and the coagulation factors V and VIII. Initially, these recations are controlled by $\alpha_2$-antiplasmin, a highly efficient inhibitor of plasmin present in plasma. As the inhibitor is depleted the systemic effects induced by plasmin become pronounced. Degradation of fibrinogen and clotting factors and accumulation of inactive fibrinogen cleavage products make blood coagulation inefficient. This might lead to serious episodes of haemorrhage. A general activation of plasminogen is therefore hazardous and should be avoided. The central issue of fibrinolytic therapy is thus to obtain an efficient plasminogen activation and fibrin removal localized at the site of occlusion, and at the same time avoiding the side effects of systemic fibrinogenolytic plasminogen activation.

Several plasminogen activators have been tested clinically for their utility as fibrinolytic agents. Streptokinase, which is a protein derived from β-hemolytic streptococci, binds to plasminogen. The complex formed is an active enzyme which converts plasminogen into plasmin. In other words, streptokinase turns plasminogen into an autoactivating enzyme. Streptokinase does not bind to fibrin clots and the systemic side effects of streptokinase ac tion are pronounced. Moreover, because streptokinase is a bacterial protein it induces a prominent immune response. Some patients possess antibodies to it, either as a consequence of previous therapy or because of past streptococcal infections. This complicates the determination of an effective therapeutic dosage.

Urokinase is a serine protease occurring naturally in human urine. It is an efficient activator of plasminogen. However, urokinase activity is not dependent on the binding to fibrin and specificity for clot resolution in vivo or in vitro has not been demonstrated. It seems to reproduce many of the systemic side effects known from streptokinase.

Prourokinase is the proenzyme form of urokinase, in which the two chains of urokinase are still joined by a peptide bond. This molecule is essentially devoid of enzymatic activity but it may have increased clot specificity relative to urokinase itself. Presumably, this arises because the plasmin catalyzed conversion to active urokinase occurs primarily on the clot. The mechanism is unclear, since prourokinase per se seems to have a low affinity for fibrin. Any manifest clinical benefit of the molecule has not been demonstrated.

Tissue plasminogen activator (t-PA) is also a serine protease, which under natural circumstances activates plasminogen. This activator differs from streptokinase and urokinase by its much stronger affinity to fibrin. Furthermore, the presence of fibrin will cause an up to 1000-fold increase in the plasminogen activation rate indicating the formation of a ternary activation complex between t-PA, plasminogen and fibrin. t-PA is a 65,000 dalton glycoprotein, which exists in two major forms: a one-chain molecule, and a two-chain molecule in which the two chains are held together by a single disulfide bond. These chains are designated the A-chain for the polypeptide originating from the N-terminal part of the enzyme, and the B-chain for the polypeptide from the C-terminal part of the enzyme. The cDNA derived amino acid sequence was first reported by Pennica et al. (Nature 301, 1983, pp. 214-221). The A-chain (the heavy chain) contains a so-called finger domain, growth factor-like domain and two kringle structures (K1 and K2) and the B-chain (the light chain) contains the active site for serine protease activity. N-terminal processing differences have been reported (Wallén et al., Eur. J.

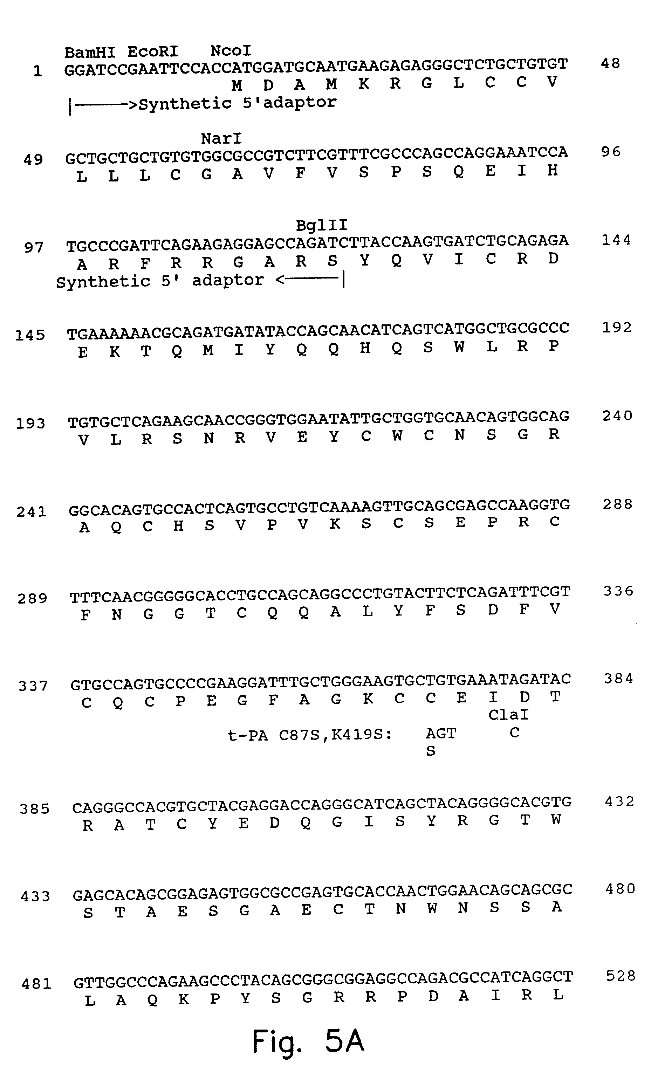

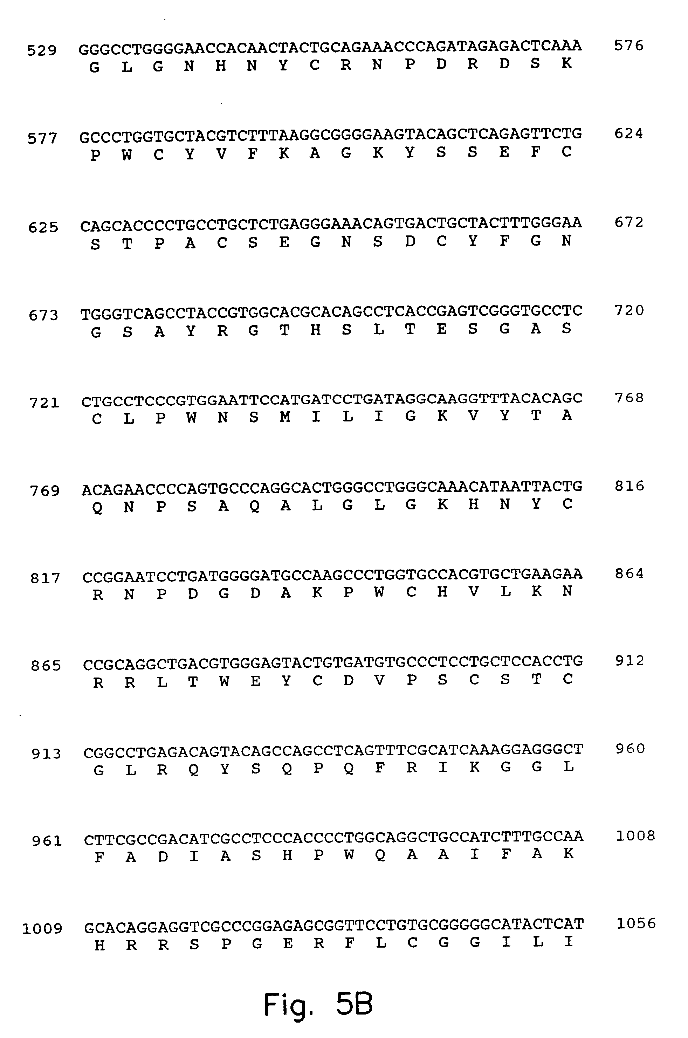

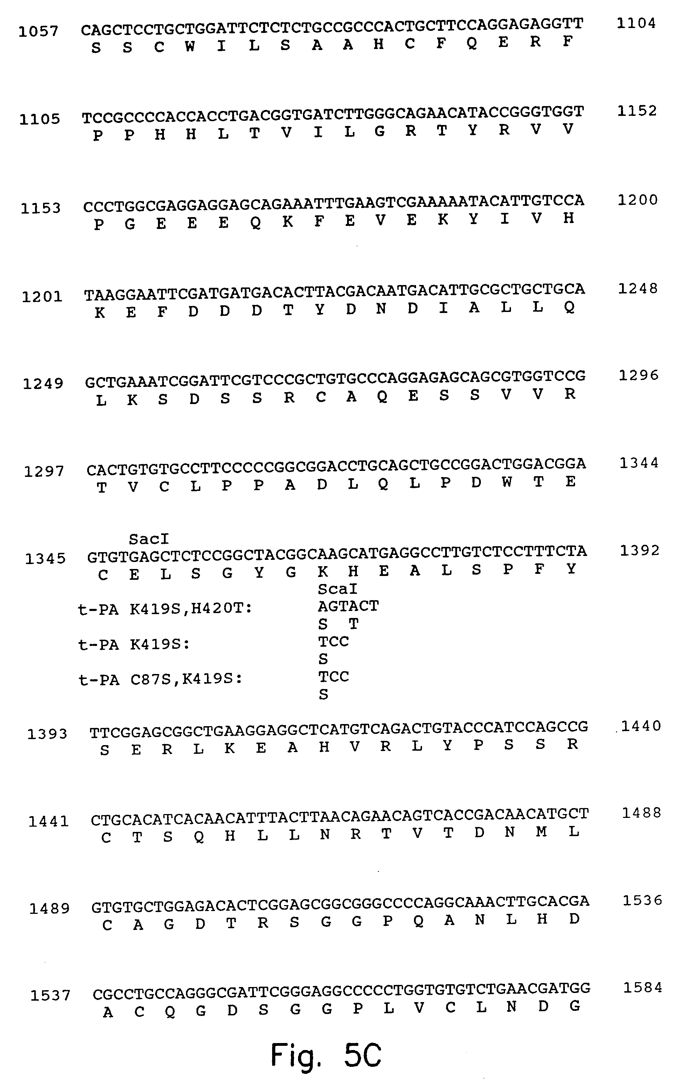

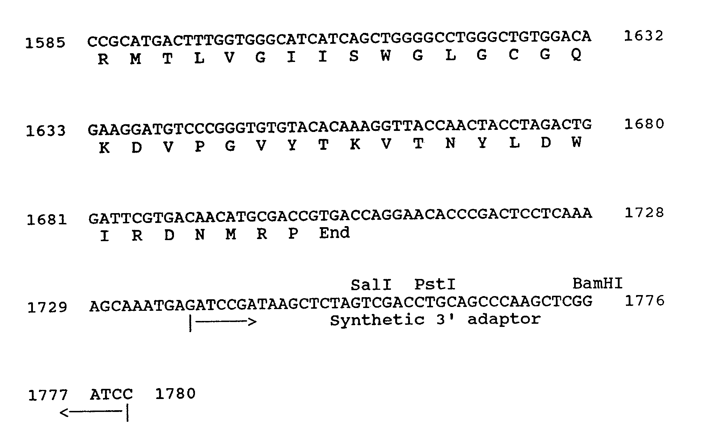

Biochem. 132, 1983, pp. 681-686) differing by the absence or presence of three additional residues. In the following, the longer of these sequences is referred to for the numbering of full-length t-PA (cf. Fig. 5A-D below).

Conversion of one-chain to two-chain t-PA is catalyzed by plasmin and consists in cleavage of a single peptide bond between the amino acids Arg(278) and Ile(279). This cleavage occurs efficiently on the surface of fibrin where plasmin is present. Both forms of the molecule are enzymatically active, but the two-chain form has a somewhat higher specific activity. The factor of activation upon conversion to the two-chain form varies with the assay system.

Due to the ability of fibrin to bind t-PA and to strongly stimulate activation of plasminogen by t-PA it was anticipated that the use of t-PA in thrombolytic treatment would eliminate the problems arising from systemic activation of plasminogen. However, elaborate clinical trials have shown that the very high dosage needed to obtain significant thrombolysis often results in systemic side effects. These effects which are induced by administration of large amounts of t-PA are presumably associated with an appreciable activity of the one-chain form even in the absence of fibrin. It would accordingly be desirable to reduce this activity and at the same time preserve the full fibrinolytic potential of two-chain t-PA when activated on the clot. The purpose of the present invention is to provide a plasminogen activator characterized by substantially reduced systemic side effects and a predominantly local lysis effect on fibrin deposits.

As mentioned above, t-PA belongs to the serine protease family. Generally these enzymes are synthesized and secreted by the cells as inactive one-chain zymogen forms. They become active enzymes only when subjected to limited proteolysis at a specific site. As exemplified by chymotrypsinogen activation, this occurs upon cleavage when the positively charged $\alpha$-amino group of Ile(16) is released to interact with Asp(194) adjacent to Ser(195) of the active site. Such salt bridge interaction appears to be necessary for establishing a proper conformation of the active site for proteolytic activity. The activation of most serine proteases applies to this general scheme and the one-chain forms of most serine proteases are genuine proenzymes. t-PA, however, possessing significant activity in the one-chain form is an exception to this rule.

It has been suggested that the active conformation of one-chain t-PA could be established as a result of an alternative salt bridge involving a properly placed amino acid residue which could provide the positively charged group even when the Arg(278)-Ile(279) bond remains intact. Whether this interaction involves the $\epsilon$-amino group of Lys(280) as proposed by Wallén (Wallén, P., Pohl, G., Bergsdorf, W., Rhånby, M., NY, T., and Jörnvall, H. (1983) Eur.J.Biochem 132. 681-686) or it involves still another residue remains to be seen.

The present invention is based on the recognition that the activity of one-chain t-PA may result from a salt bridge interaction between the $\epsilon$-amino group of the lysine residue in position 419 and the $\beta$-carboxy group of the aspartic acid residue in position 480. The putative presence of a salt bridge between these lysine and aspartic acid residues, predicted on the basis of a computer-graphical model of the protease domain of the t-PA molecule, is tentatively suggested as the reason for the activity of one-chain t-PA (A. Heckel and K.M. Hasselbach, J. Computer-Aided Mol. Design 2, 1988, pp. 7-14). There is, however, no indication that the one-chain form of t-PA may be inactivated by preventing salt bridge formation at this site, while substantially retaining the activity of the two-chain form of t-PA.

SUMMARY OF THE INVENTION

The present invention relates to a tissue plasminogen activator (t-PA) analogue in substantially inactivated one-chain form capable of being converted to the active two-chain form, which analogue is modified in the region from position 414 to position 419 or from position 419 to position 426 or from position 426 to position 433.

It is currently believed that a salt bridge may be formed between the aspartic acid residue in position 480 and an amino acid residue within a distance of about 8 Å from Asp(480) in the three-dimensional structure of t-PA. This is the case with the amino acid residues from position 414 to position 433 in the t-PA molecule. Theoretically, therefore, each of these residues represent a possible site of salt bridge interaction with Asp(480).

More specifically, however, the invention relates to a t-PA analogue in which the interaction of a positively charged amino acid residue in position 419 and optionally also in position 420 with a negatively charged amino acid residue in position 480 is inhibited.

Thus, the present invention surprisingly provides a t-PA analogue which, in the one-chain form, exhibits the properties of a proenzyme (i.e. it is substantially inactive), whereas the activity of the two-chain form is fully retained on plasmin-catalysed cleavage of the one-chain t-PA analogue. Compared to authentic (native) t-PA, this results in a fibrinolytic agent with a higher fibrin selectivity as the fibrinogenolytic activity induced by the t-PA analogue of the invention is substantially reduced relative to that induced by native one-chain t-PA.

In the present context, the term "substantially inactivated" is intended to indicate that the t-PA analogue of the invention exhibits little or no catalytic activity in the one-chain form with respect to the amidolytic activity on simple synthetic substrates as well as the plasminogen activating activity.

The amidolytic activity, i.e. the general catalytic activity, may for instance be measured by means of an assay involving the use of a chromogenic low molecular weight substrate such as D-Ile-Pro-Arg-pNA (S-2288) (Kabi, Stockholm, Sweden) which is cleaved directly by t-PA and therefore offers a convenient colorimetric determination of the amidolytic activity (Petersen et al., Biochim. Biophys. Acta 952, 1988, pp. 245-254).

The plasminogen activating activity may for instance be determined indirectly in an assay involving the use of plasminogen and a chromogenic substrate such as D-Val-Phe-Lys-pNA (S-2390) (Kabi, Stockholm, Sweden) which is cleaved by plasmin. In this assay, colour development fol lows an accelerating parabolic curve as plasmin accumulates. The acceleration constant is directly proportional to the activity of t-PA. As this assay determines the proteolytic activity of t-PA on the natural substrate plasminogen, it is more closely related to the fibrinolytic effect (Petersen et al., op.cit.). In both types of assay, it is possible to include fibrin so as to study its stimulating effect on the t-PA enzymatic activity.

DETAILED DISCLOSURE OF THE INVENTION

In one preferred embodiment of the present invention, the t-PA analogue is one which has no positively charged amino acid in position 419. This may be effected by deleting Lys(419) from the native t-PA molecule. However, as deletion may possibly lead to incorrect folding of the resulting protein which in turn may lead to inactivation of the two-chain form of t-PA as well, it is currently preferred to replace Lys(419) of native t-PA by a negatively charged or uncharged amino acid residue. Examples of suitable amino acid residues are Ser, Thr, Glu, Val, Ala, Leu, Ile, Tyr, Asn, Asp and Gln. Substitution by for instance Ser or Lys may possibly be preferred in order to avoid too bulky groups in the molecule resulting in insufficient or incorrect folding.

The t-PA analogue carrying a substitution in position 419 may further be modified in position 420, preferably by replacing a positively charged amino acid residue in position 420 by a negatively charged or uncharged amino acid residue. Thus, in a particular embodiment, His(420) may be replaced by an amino acid residue selected from the group consisting of Ser, Thr, Glu, Val, Ala, Leu, Ile, Tyr, Asn, Asp and Gln.

Although it may theoretically be possible to perform a similar substitution of the negatively charged amino acid residue in position 480 with a positively charged or uncharged amino acid residue rather than substituting Lys(419) and optionally His(420), this is currently believed to be less desirable as such substitution may interfere with the serine protease active site of t-PA and thereby result in inactivation of the two-chain form of t-PA.

T-PA analogues according to the present invention may additionally comprise a modification in the A-chain, always provided that such a further modification has no negative effect on the characteristics of the t-PA analogue of the invention, especially with respect to its plasminogen activating capability in the two-chain form. Such modifications are well known and include deletions or modifications of the finger domain and/or growth factor sequence, deletion or modification of kringle 1 or kringle 2 and/or replacement of kringle 1 of t-PA by kringle 1 of plasminogen; cf. Ep 196 920, EP 207 589, EP 231 624, DK 3022/88 and DK 3023/88.

In another aspect, the present invention relates to a DNA construct which comprises a DNA sequence encoding a t-PA analogue which is modified in the region from position 414 to position 419 or from position 419 to position 426 or from position 426 to position 433.

More preferably, a codon specifying a positively charged amino acid in position 419 of the t-PA analogue encoded by the DNA sequence has been replaced by another codon specifying a negatively charged or uncharged amino acid. Optionally, a codon specifying a positively charged amino acid in position 420 of the t-PA analogue encoded by the DNA sequence has additionally been replaced by another codon specifying a negatively charged or uncharged amino acid.

The DNA construct of the invention encoding the t-PA analogue of the invention may be prepared synthetically by established standard methods, e.g. the phosphoamidite method described by S.L. Beaucage and M.H. Caruthers, Tetrahedron Letters 22, 1981, pp. 1859-1869, or the method described by Matthes et al., EMBO Journal 3, 1984, pp. 801-805. According to the phosphoamidite method, oligonucleotides are synthesized, e.g. in an automatic DNA synthesizer, purified, duplexed and annealed.

The DNA construct of the invention may also be of genomic or cDNA origin, for instance obtained by preparing a genomic or cDNA library and screening for DNA sequences coding for all or part of the polypeptide of the invention by hybridization using synthetic oligonucleotide probes in accordance with standard techniques (cf. T. Maniatis et al., Molecular Cloning: A Laboratory Manual, Cold Spring Harbor, 1982). In this case, a genomic or cDNA sequence encoding native human t-PA may be modified at a site corresponding to the site(s) at which it is desired to introduce amino acid substitutions, e.g. by site-directed mutagenesis using synthetic oligonucleotides encoding the desired amino acid sequence for homologous recombination in accordance with well-known procedures.

Finally, the DNA construct may be of mixed synthetic and genomic, mixed synthetic and cDNA or mixed genomic and cDNA origin prepared by annealing fragments of synthetic, genomic or cDNA origin (as appropriate), the fragments corresponding to various parts of the entire DNA construct, in accordance with standard techniques.

A preferred DNA construct of the invention has a DNA sequence substantially as shown in the appended Fig. 5 A-D, or a suitable modification thereof. The modification may constitute any one of the known modifications of t-PA, such as one of the modifications mentioned above. The modification may also be one which involves the substitution of one or more nucleotides in the DNA sequence by one or more others which do not give rise to an altered amino acid sequence of the t-PA analogue, but which, for instance, provide a convenient restriction enzyme cleavage site in the DNA sequence encoding the analogue.

In a further aspect, the present invention relates to a recombinant expression vector carrying a DNA sequence encoding the t-PA analogue of the invention may be any vector which may conveniently be subjected to recombinant DNA procedures, and the choice of vector will often depend on the host cell into which it is to be introduced. Thus, the vector may be an autonomously replicating vector, i.e. a vector a vector which exists as an extrachromosomal entity, the replication of which is independent of chromosomal replication, e.g. a plasmid. Alternatively, the vector may be one which, when introduced into a host cell, is integrated into the host cell genome and replicated together with the chromosome(s) into which it has been integrated.

In the vector, the DNA sequence encoding the t-PA analogue of the invention should be operably connected to a suitable promoter sequence. The promoter may be any DNA sequence which shows transcriptional activity in the host cell of choice and may be derived from genes encoding proteins either homologous or heterologous to the host cell. Examples of suitable promoters for directing the transcription of the DNA encoding the t-PA analogue of the invention are the SV 40 promoter (Subramani et al., Mol. Cell Biol. 1, 1981, pp. 854-864), the MT-1 (metallothionein gene) promoter (Palmiter et al., Science 222, 1983, pp. 809-814) or the adenovirus 2 major late promoter.

The DNA sequence encoding the t-PA analogue of the invention should also be operably connected to a suitable transcriptional terminator, such as the human growth hormone gene terminator (Palmiter et al., op.cit.). The vector should further comprise elements such as polyadenylation signals (e.g. from SV 40 or the adenovirus 5 Elb region), transcriptional enhancer sequences (e.g. the SV 40 enhancer) and translational enhancer sequences (e.g. the ones encoding adenovirus VA RNAs).

The recombinant expression vector of the invention further comprises a DNA sequence enabling the vector to replicate in the host cell in question. An examples of such a sequence (when the host cell is a mammalian cell) is the SV 40 origin of replication. The vector may also comprise a selectable marker, e.g. a gene the product of which complements a defect in the host cell, such as the gene coding for dihydrofolate reductase (DHFR) or one which confers resistance to a drug, e.g. neomycin, hygromycin or methotrexate.

The procedures used to ligate the DNA sequences coding for the t-PA analogue of the invention, the promoter and the terminator, respectively, and to insert them into suitable vectors containing the information necessary for replication, are well known to persons skilled in the art (cf., for instance, Maniatis et al., op.cit.).

In a still further aspect, the present invention relates to a host cell transformed or transfected with the recombinant expression vector described above. In the present context, the term "transformed" is used when the vector is a plasmid vector functional in a bacterial, yeast or fun gal cell, while the term "transfected" is used when the vector is a viral vector, i.e. one containing a viral origin of replication.

The invention also relates to a method of preparing a t-PA analogue in inactivated one-chain form, the method comprising

(a) modifying a DNA sequence encoding native t-PA by replacing a codon specifying a positively charged amino acid in position 419 of native t-PA by a negatively charged or uncharged amino acid,

(b) inserting the DNA sequence prepared in step (a) into a suitable replicable expression vector,

(c) transforming or transfecting a suitable host cell with the expression vector prepared in step (b), and

(d) growing the transformed host cell in a suitable growth medium under conditions conducive to the expression of the t-PA analogue, and recovering the analogue from the culture.

Optionally, a codon specifying a positively charged amino acid in position 420 of the native protein may additionally be replaced by a codon specifying a negatively charged or uncharged/neutral amino acid in step (a) of the method of the invention.

Alternatively, the t-PA analogue may be prepared by producing authentic t-PA by recombinant DNA techniques as described above and subjecting the t-PA produced to suitable chemical treatment to convert it to the t-PA analogue of the invention.

The host cell may be any cell which is capable of producing t-PA, and is typically a mammalian cell. Examples of suitable mammalian cell lines are the COS (ATCC CRL 1650), BHK (ATCC CRL 1632, ATCC CCL 10) or CHO (ATCC CCL 61) cell lines. Methods of transfecting mammalian cells and expressing DNA sequences introduced in the cells are described in e.g. Kaufman and Sharp, J. Mol. Biol. 159, 1982, pp. 601-621; Southern and Berg, J. Mol. Appl. Genet. 1, 1982, pp. 327-341; Loyter et al., Proc. Natl. Acad. Sci. USA 79, 1982, pp. 422-426; Wigler et al., Cell 14, 1978, p. 725; Corsaro and Pearson, Somatic Cell Genetics 7, 1981, p. 603, Graham and van der Eb, Virology 52, 1973, p. 456; and Neumann et al., EMBO J. 1, 1982, pp. 841-845. The medium used to cultivate the cells may be any conventional medium suitable for growing mammalian cells, such as a serum-containing or serum-free medium containing appropriate supplements. Suitable media are available from commercial suppliers or may be prepared according to published recipes (e.g. catalogues of the American Type Culture Collection).

The t-PA analogue produced by the cells will typically be secreted into the growth medium and may be recovered from the medium by conventional procedures including separating the host cells from the medium by centrifugation or filtration, precipitating the proteinaceous components of the supernatant or filtrate by means of a salt, e.g. ammonium sulphate, followed by purification by a variety of chromatographic procedures, e.g. ion exchange chromatography, affinity chromatography, or the like.

In a yet further aspect, the present invention relates to a pharmaceutical composition which comprises a t-PA analogue of the invention and a pharmaceutically acceptable diluent or vehicle. Before use, the t-PA analogue may be stored in dry, e.g. lyophilized, form and be formulated for parenteral administration (e.g. for injection or infusion) by being dissolved or suspended in an appropriate liquid vehicle such as sterile isotonic saline. The dosage of the t-PA analogue to be administered is expected to be in the same range as that employed for other t-PA-containing compositions, i.e. about 10-200 mg.

The invention also relates to the use of a t-PA analogue of the invention for the preparation of a medicament for the treatment of diseases or disorders associated with the formation of thrombi in blood vessels. Examples of such diseases or disorders are infarctions, thrombosis and embolism.

BRIEF DESCRIPTION OF THE DRAWINGS

The invention is further described with reference to the drawings in which

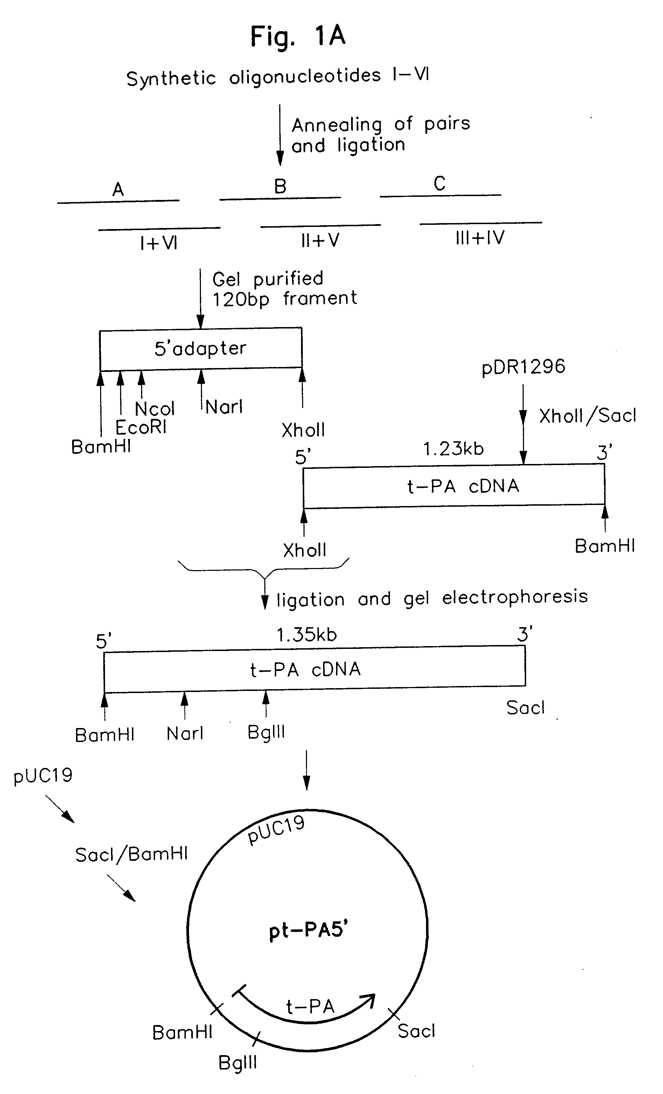

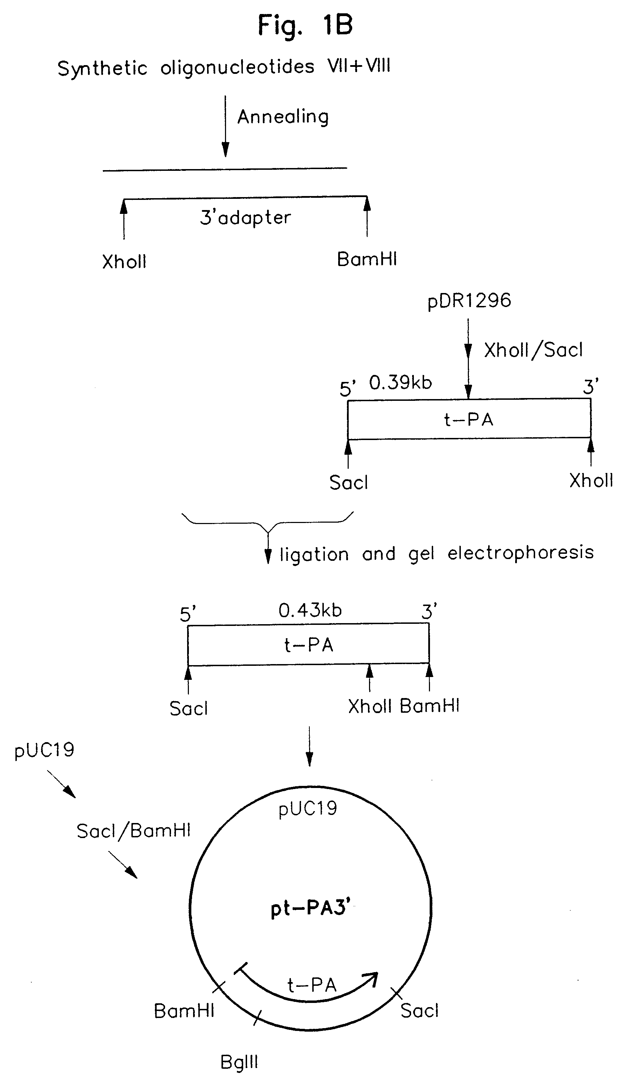

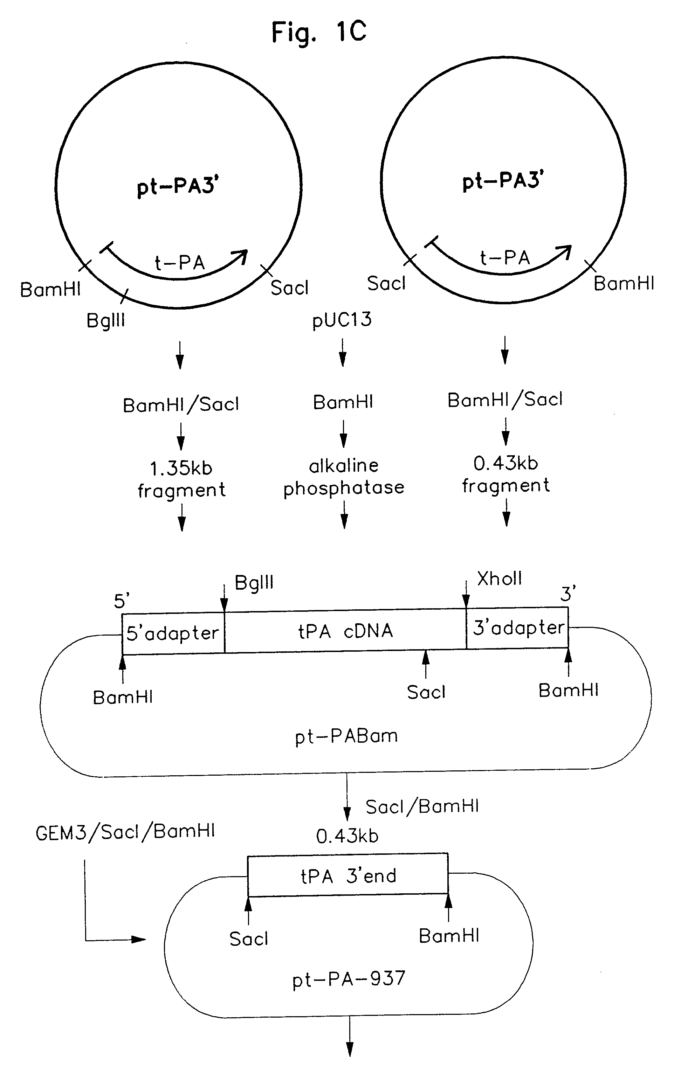

Fig. 1A-C shows the step-wise construction of plasmids pt-PABam and pt-PA-937,

Fig. 2 shows the construction of plasmid pt-PA-[K419S,H420T] (formerly pBoel-1079) from plasmid pt-PABam and pt-PA-937 and the synthetic oligonucleotide NOR 518,

Fig. 3 shows the construction of plasmid pt-PA[K419S] from plasmids pt-PABam and pt-PA-937 and the synthetic oligonucleotide NOR 517,

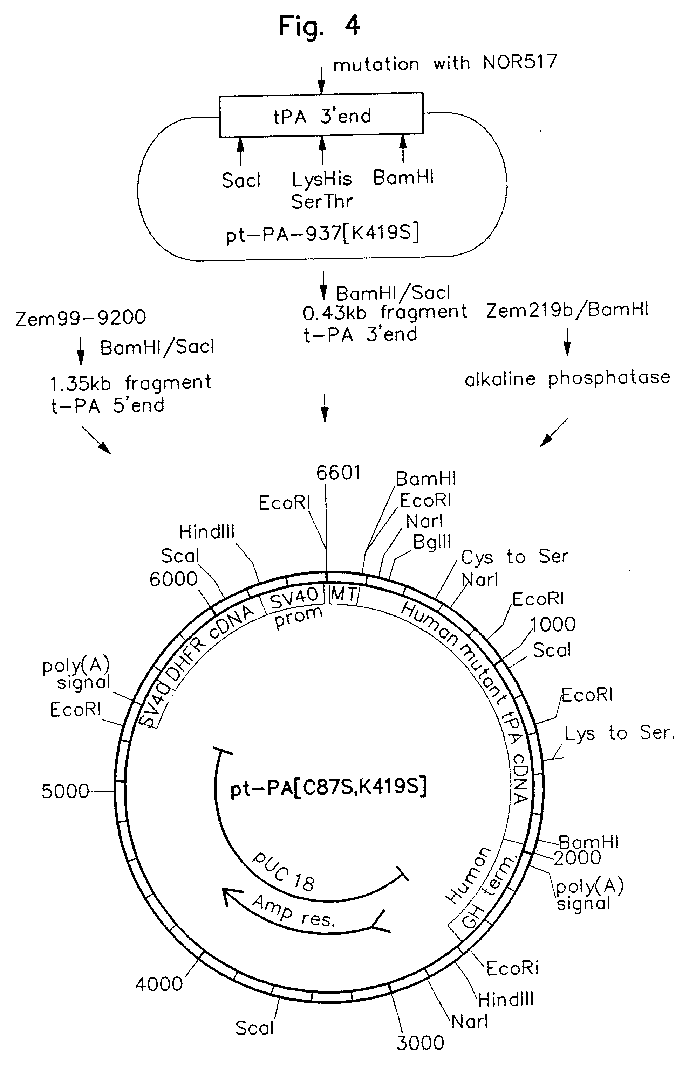

Fig. 4 shows the construction of plasmid pt-PA[C87S,K419S] from plasmids Zem99-9200 and pt-PA[K419S],

Fig. 5 A-D shows the DNA sequence together with the encoded amino acid sequence of a 1.78 kb t-PA BamHI fragment of plasmid pt-PABam; the 5' and 3' synthetic adapters are indicated with horizontal arrows, and the nucleotide substitutions leading to the amino acid substitutions C87S, K419S, H420T, and K419S are indicated below the wild-type amino acid sequence,

Fig. 6 A and B shows the amidolytic activity of authentic t-PA and t-PA[K419S,H420T] before and after plasmin cleavage (Δ denotes native one-chain t-PA, o denotes native two-chain t-PA, ▲ denotes one-chain t-PA[K419S,H420T], ● denotes two-chain t-PA[K419S,H420T], and □ denotes one-chain t-PA[K419S,H420T] in the presence of 100 μM transaminomethyl cyclohexane 1-carboxylic acid)

Fig. 7 shows the neutralization of α-antiplasmin activity induced by authentic t-PA and t-PA[K419S,H420T] in human plasma (Δ denotes authentic t-PA, and o denotes t-PA [K419S,H420T]), and

Fig. 8 shows the thrombolytic effect of authentic t-PA (●) and t-PA[K419S,H420T] (o) measured by means of an artery-venous shunt in anesthetized rabbits.

The invention is further described in the following examples which are not in any way intended to limit the scope or spirit of the invention as claimed.

Example 1

I. Construction of a Full-Length t-PA cDNA Clone.

The sequence of a native human t-PA cDNA clone has previously been reported (Pennica et al., Nature 301: 214-221, 1983). The sequence encodes a pre-pro peptide of 32-35 amino acids followed by a 527-530 amino acid mature protein.

A cDNA clone comprising the coding sequence for mature t- PA was constructed using mRNA from the Bowes melanoma cell line (Rijken and Collen, J. Biol. Chem. 226: 7035 - 7041, 1981) as the starting material. This cDNA was then used to construct the plasmid pDR1296. Escherichia coli strain JM83 transformed with pDR1296 was deposited on 10th December 1985 in the American Type Culture Collection, Rockville, Maryland, USA, with the Accession No. 53347.

The coding region for the complete t-PA pre-pro sequence was not present in the isolated cDNA clone pDR1296, and the DNA encoding this region was therefore constructed from synthetic oligodeoxyribonucleotides and subsequently ligated as an adapter to the cloned cDNA. In this synthetic adapter, cleavage sites for BamHI, EcoRI and Ncol were introduced immediately 5' to the first codon (ATG) of the pre-pro sequence. The naturally occurring pre-pro sequence lacks a convenient restriction site near the middle, for which reason the sequence GGAGCA (coding for amino acids 20 and 19, Gly-Ala) was altered to GGCGCC to provide a Narl site without changing the amino acid sequence.

Likewise the 3' end of the cloned t-PA cDNA was joined to an adapter sequence (with a BamHI site at the 3' end) made up of synthetic oligodeoxyribonucleotides. Synthesis of all oligonucleotides was performed on an Applied Bio-systems Model 380-A DNA synthesizer using phosphoramidite chemistry on a controlled pore glass support (Beaucage and Caruthers, Tetrahedron Letters 22: 1859 - 1869, 1981). The following 8 oligonucleotides were synthesized:

5' adapter:

I:      GATCCGAATTCCACCATGGATGCAATGAAGAGAGGGCTCTGCTG

44-mer

II:      TGTGCTGCTGCTGTGTGGCGCCGTCTTCGTTTCGCCCAGCCAGGA

45-mer

III: AATCCATGCCCGATTCAGAAGAGGAGCCA

29-mer

IV: GATCTGGCTCCTCTTCTGAATCGGGCATGGATTTCCTGGCTGG

43-mer

V: GCGAAACGAAGACGGCGCCACACAGCAGCAGCACACAGCAGAGCCC

46-mer

VI: TCTCTTCATTGCATCCATGGTGGAATTCG

29-mer

3' adapter:

VII: GATCCGATAAGCTCTAGTCGACCTGCAGCCCAAGCTCG

38-mer

VIII: GATCCGAGCTTGGGCTGCAGGTCGACTAGAGCTTATCG

38-mer

20 pmol of each of the corresponding pairs A - D of 5'-phosphorylated oligonucleotides (A: I and VI; B: II and V; C: III and IV; D: VII and VIII) were heated for 5 min. at 90°C followed by cooling to room temperature over a period of 75 minutes. Pairs A, B and C (5' adapter) were mixed and treated with $T_4$ DNA ligase, and the ligated synthetic adapter was isolated as a 120 bp fragment after electrophoresis of the ligation mixture on a 2 % agarose gel. This 5' BamHI/XhoII adapter was ligated to the 5' 1.23 kb XhoII/SacI fragment of the t-PA cDNA from plasmid pDR1296; the ligation products were separated on a 1 % agarose gel and a 1.35 kb fragment was eluted and cloned in a BamHI/SacI digested pUC19 vector to generate plasmid pt-PA5'. Restriction enzyme digestions with BglII on isolated plasmids were used to identify correct DNA constructs. Correct ligation of the two XhoII sites generated a BglII site at the junction between the 5' 120 bp adapter and the 5' 1.23 kb t-PA fragment.

The 3' 0.39 kb SacI/XhoII t-PA fragment from the t-PA cDNA clone pDR1296 was ligated to oligonucleotide pair D, and the ligation products were separated on a 5 % polyacrylamide gel. A 0.43 kb fragment was eluted and cloned in a BamHI/SacI digested pUC19 vector to generate plasmid pt-PA3'. Restriction enzyme digestions on isolated plasmids were used to identify correct DNA constructs.

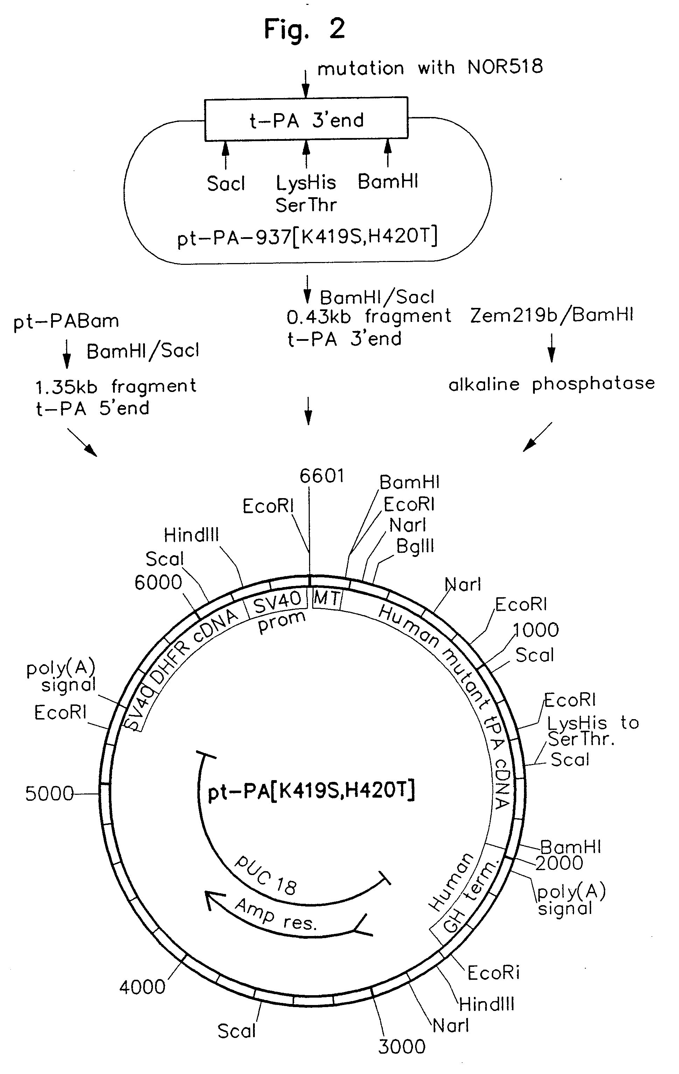

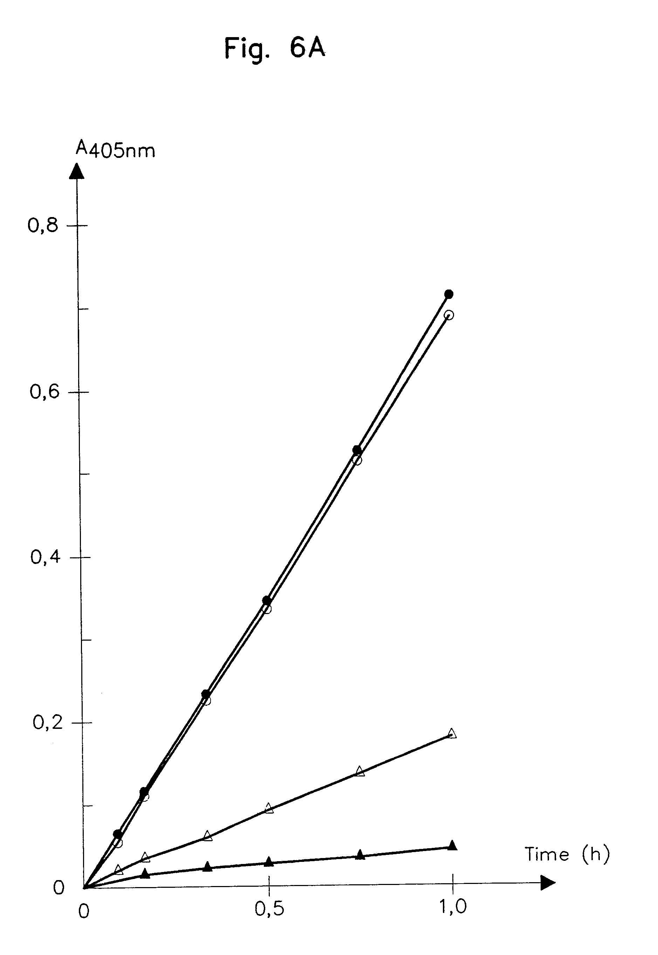

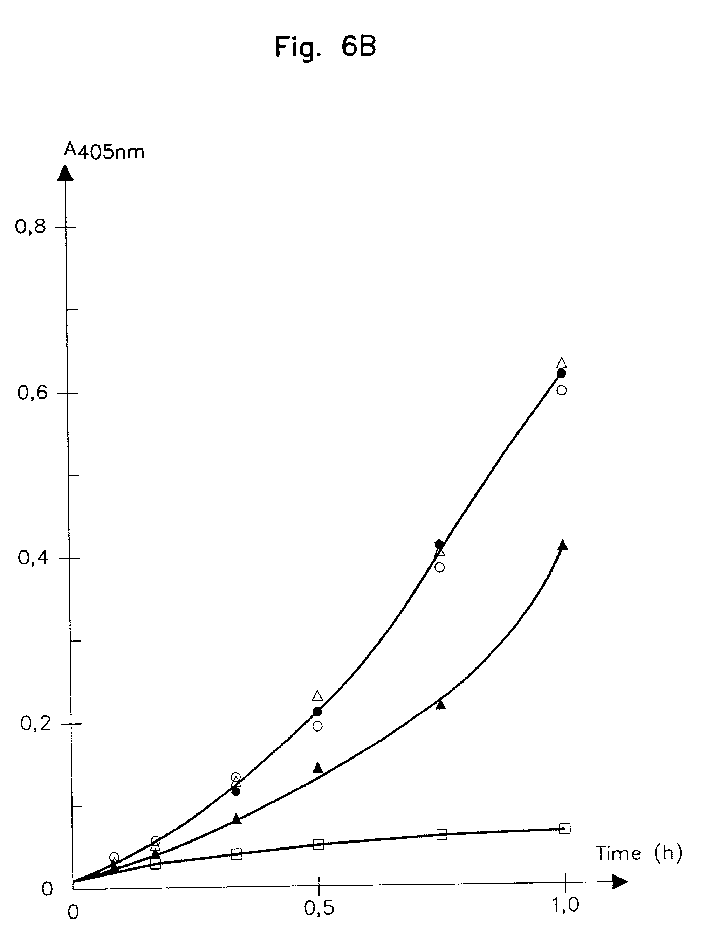

Finally the full-length prepro-t-PA cDNA with its 5' and 3' flanking synthetic adapters was assembled by ligation of the 1.35 kb 5' t-PA fragment from pt-PA5' and the 0.43 kb t-PA fragment from pt-PA3' with a BamHI digested and alkaline phosphatase- treated pUC13 vector to produce plasmid pt-PABam. The sequence of the resulting 1.78 kb t-PA BamHI fragment in ptPABam is shown in figure 6. The DNA sequence of the prepro-t-PA cDNA was confirmed by subcloning and sequencing of DNA fragments in M13 vectors. In figure 2 all synthetic DNA (5' and 3' adapter fragments) is indicated with horizontal arrows.

II. Mutation of t-PA cDNA to substitute Ser for Lys in position 419 and Thr for His in position 420.

From ptPABam a 0.43 kb SacI/BamHI fragment, which contained the 3' end of the t-PA cDNA was isolated. It was ligated to a SacI/BamHI digested GEM3 vector (Promega Biotec.), and the resulting plasmid pt-PA-937 was used in mutagenesis experiments in E. coli as described by Morinaga, Y., et al (BIO/TECHNOLOGY, 2: 636 - 639, 1984). Two mutagenic oligonucleotides, the 31'mer (NOR 518):
5'(AGACAAGGCCTCAGTACTGCCGTAGCCGGAG)3'
and the 31'mer (NOR 517):
5'(AGACAAGGCCTCATGGGAGCCGTAGCCGGAG)3'
were used. NOR 518 has four mismatches to human wild-type t-PA cDNA in the region encoding the amino acid sequence Ser-Gly-Tyr-Gly-Lys-His-Glu-Ala-Leu-Ser (residues no. 447 - 456 of the t-PA precursor). After in vitro mutagenesis the sequence reads Ser-Gly-Tyr-Gly-Ser-Thr-Glu-Ala-Leu-Ser, and a mutation Lys-His to Ser-Thr has been made (K419S, H420T). This mutation creates a new ScaI restriction site in the cDNA sequence. NOR 517 has three mismatches to human wild type t-PA cDNA in the region encoding the amino acid sequence Ser-Gly-Tyr-Gly-Lys-His-Glu-Ala-Leu-Ser (residues no. 447 - 456 of the t-PA precursor). After in vitro mutagenesis the sequence reads Ser-Gly-Tyr-Gly-Ser-His-Gly-Ala-Leu-Ser, and a mutation Lys to Ser has been made (K419S).

The mutant genotypes were identified by colony hybridizations with $^{32}$P labelled mutagenic oligonucleotides NOR 518 and NOR 517, and positive clones were sequenced to verify the mutations.

From the resulting mutant plasmids: pt-PA-937[K419S,H420T] and pt-PA-937[K419S], the 0.43 kb SacI/BamHI fragments were isolated and ligated to the 5' end of human t-PA cDNA (as a gel-purified 1.35 kb

BamHl/Sacl fragment from pt-PABam) in the BamHl digested mammalian expression vector Zem219b (described in DK patent application No. 3023/88). Two 6.6 kb plasmids pt-PA[K419S,H420T] and pt-PA[K419S] with the mutant t-PA cDNAs inserted in the correct orientation, were identified by restriction enzyme digestion.

The 5′ end of the t-PA mutant 9200 (described in DK patent application No. 3023/88) was isolated as a BamHl/Sacl fragment from the plasmid Zem99-9200 described therein, and ligated to the 0.43 kb Sacl/BamHl fragment from pt-PA-937[K419S] in BamHl digested Zem219b to generate pt-PA[C87S,K419S]. From this construction a t-PA mutant may be produced, in which the longer half-life of the t-PA mu- tant 9200 is combined with the low fibrinogenolytic activity of t-PA[K419S].

### III. Expression of mutant t-PA cDNAs in mammalian cells.

For expression of mutant t-PA in cultured BHK cells (Syrian Hamster, thymidine kinase mutant line tk⁻ts13, Waechter and Baserga, Proc. Natl. Acad. Sci. USA, 79: 1106 - 1110, 1982. American Type Culture Collection CRL 1632) expression vectors pt-pA[K419S,H420T], pt-pA[C87S,K419S] and pt-PA[K419S] were introduced in three separate experiments into cells by the calcium phosphate-mediated transfection procedure (Graham and Van der Eb, Virology, 52: 456 - 467, 1973). 48 hrs after transfection, cells were trypsinized and diluted into medium containing 400 nM methotrexate (MTX). After 10 to 12 days, individual colonies from each of the three experiments were cloned out and expanded separately. Screening for t-PA mutant production was done by an enzyme-linked immunosorbent assay (ELISA) with monoclonal antibodies against wild-type human t-PA.

### IV. Purification of t-PA analogues from cell culture supernatants.

The human one-chain tissue plasminogen activator analogues t-PA[K419S], t-PA[K419S,H420T] and t-PA[C87S,K419S] were purified from the cell culture supernatants by immunoaffinity chromatography as previously described (Petersen, L.C., Johannessen, M., Foster D., Kumar, A. and Mulvihill, E. (1988), Biochim.Biophys.Acta. 952, 245-254). The specific activity was determined by the fibrin clot lysis assay (Gaffney, P.J., Templeman, J., Curtis, A.D. and Campbell, P.J. (1983), Thromb. Haemostas 50, 650-651). A value of 60 IU/μg was obtained for the one-chain form. This increased to 320 IU/μg when it was converted into the two-chain form by incubation with 8.8 nM plasmin for 30 minutes. The protein concentration was determined by ELISA, and the clot lysis activity was calibrated against an international reference t-PA 83/517 obtained from Dr. P.J. Gaffney (National Institute of Biological Standards and Control, London, UK). The purified t-PA analogues contained less than 2% of the two-chain form as judged from reduced SDS-PAGE. Authentic, human recombinant t-PA expressed by BHK cells was purified by a similar procedure and used for comparison.

### Example 2

### Functional properties of Lys419 substitution analogues

#### I. Intrinsic activity of one-chain Lys419 substitution analogues

The catalytic activity of one-chain t-PA analogues was studied with chromogenic peptide substrates and with plasminogen. The chromogenic substrates Ile-Pro-Arg-pNA (S 2288), and Val Phe-Lys-pNA (S 2390) and human fibrinogen and plasmin were obtained from Kabi (Stockholm, Sweden). Human Lys[77]-plasminogen was purified as previously described (Thorsen, S., Clemmensen, I., Sottrup-Jensen, L. and Magnusson, S. (1981), Biochim.Biophys.Acta. 668, 377-387). Thrombin was obtained from Leo Pharmaceutical Products (Ballerup, Denmark). The amidolytic activity with S 2288 is shown in Fig. 6A and B. The reaction mixture contained 10 nM t-PA, 1 μM aprotinin, 0.6 mM S2288, 0.1 M NaCl, 0.05 M Tris Cl, 0.01% Tween 80, pH 7.4, 25°C. The intrinsic activity of authentic one-chain t-PA with this substrate amounted to about 25% of the two-chain activity, whereas the activity of t-PA[K419S, H420T] was about 10-fold lower. Incubation with 6 nM plasmin for 30 minutes resulted in full conversion of both proteins into their two-chain forms and restored full two-chain activity also with t-PA[K419S,H420T]. The results indicated that the one-chain t-PA[K419S,H420T] possessed at most a few percent of the activity of its two-chain counterpart. It could not be excluded that this activity was fully or in part explained by two-chain contamination. In any case, one-chain t-PA[K419S,H420T] was far less active with this substrate than authentic one-chain t-PA. This was also true for the activity with the natural substrate, plasminogen, in the absence of fibrin (results not shown). Similar results were obtained with t-PA[K419S] and t-PA[C87S,K419S].

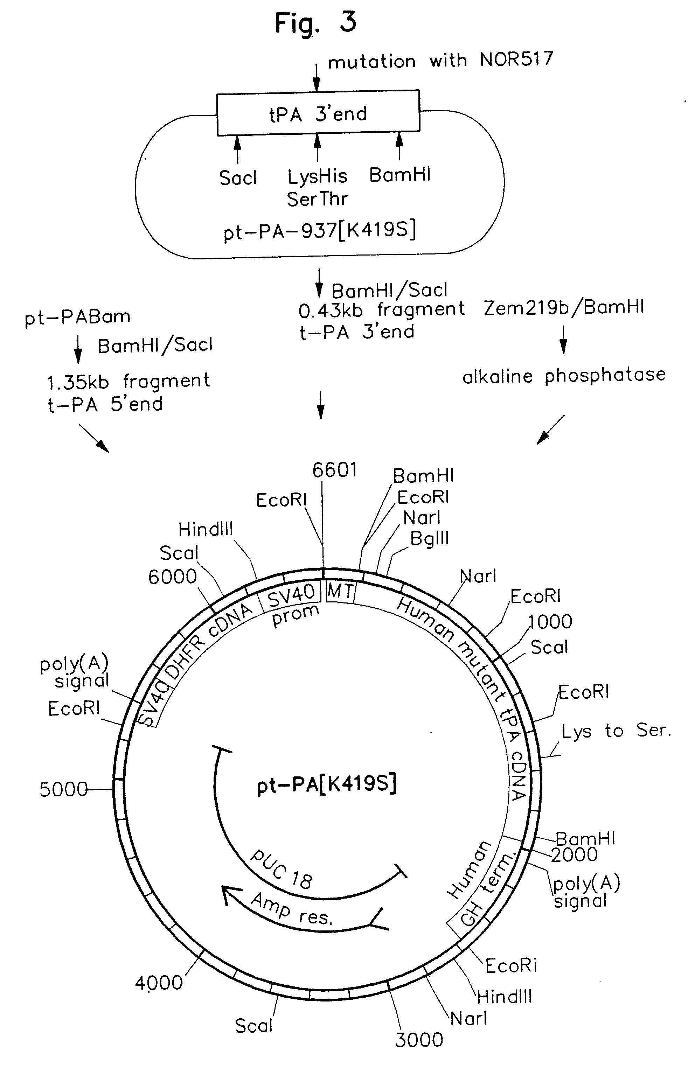

The activity with plasminogen in the presence of fibrin is shown in Fig. 3B. This was determined as previously described (Petersen et al., Biochim.Biophys.Acta 952, 245-254, 1988). The reaction mixture contained 0.06 nM t-PA, 0.2 μM Lys[77]-plasminogen, 0.6 mM S2390, 0.15 μM fibrinogen, 0.1 NIHU/ml thrombin, 0.2 μM aprotinin. Other conditions were as described above. The activity with one-chain authentic t-PA was indistinguishable from that of the two-chain form. Also the plasminogen activation with two-chain t-PA[K419S,H420T] proceeded at a similar rate in this assay. However, the activity of one-chain t-PA[K419S,H420T] was somewhat decreased, although the major effect might well be caused by a delay in the lag phase before the maximum stimulation of plasminogen activation (maximum slope of the progress curve) was reached.

II. Fibrinogenolytic and thrombolytic activity of t-PA[K419S,H420T]

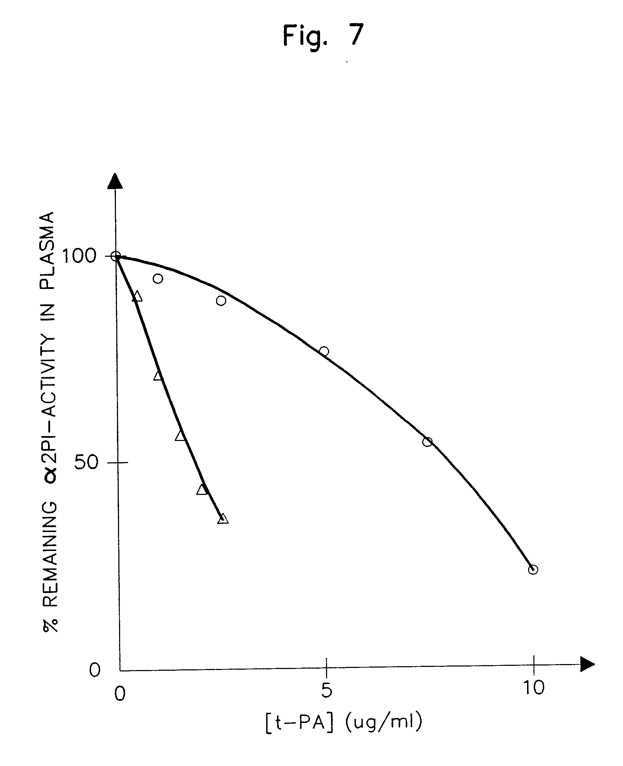

The plasminogen activation in plasma induced by authentic t-PA and t-PA[K419S,H420T] was tested by measuring the decrease in antiplasmin activity. This parameter is an indication of the systemic effects expected due to plasmin generation in the absence of a fibrin clot. Human citrate plasma was incubated for 3 hours with various amounts of t-PA as indicated. Then the plasma was diluted 10-fold in buffer containing 200 μM trans-4-(aminomethyl)cyclohexane-1-carboxylic acid and analysed for $\alpha_2$-antiplasmin activity. The plasmin inhibition capacity of a plasma control without t-PA was set to 100%. The results show (Fig. 7) that non-specific plasmin generation with authentic t-PA was obtained at a much lower dose than with t-PA[K419S,H420T]. A 50% decrease in antiplasmin over 3 hrs was observed with 1.9 μg/ml t-PA whereas a similar decrease was only observed with 7.5 μg/ml t-PA-1079. This indicates that systemic, fibrinogenolytic effects caused by Lys419 substitution analogues are much less pronounced than with authentic t-PA.

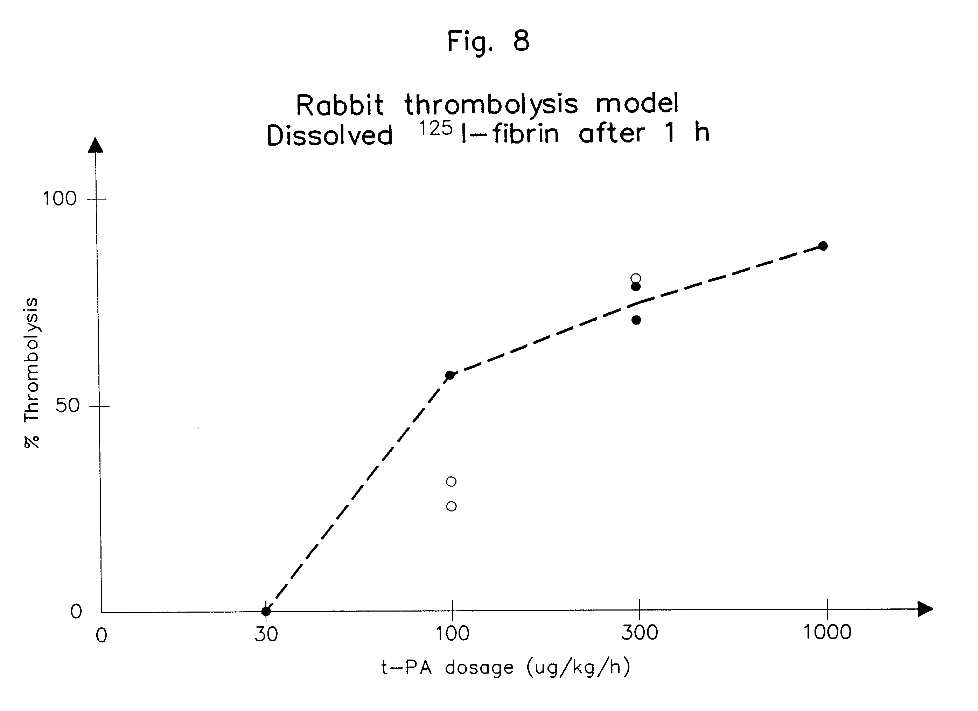

The fibrinolytic efficiency in vivo was tested in a rabbit model with an external shunt from the carotid artery to the jugular vein. A pre-formed $^{125}$I-fibrinogen-containing whole blood thrombus was placed in the shunt and t-PA infusion was initiated. Various amounts of t-PA per kg body weight was infused via an ear vein over one hour and the $^{125}$I-fibrinogen content of the thrombus was monitored with a gamma-counter. As shown in Fig. 8 the t-PA[K419S,H420T] analogue was as efficient as authentic t-PA in dissolving the thrombus.

The features disclosed in the foregoing description, in the following Claims and/or in the accompanying drawings may, both separately and in any combination thereof, be material for realising the invention in diverse forms thereof.

## Claims

1. A tissue plasminogen activator (t-PA) analogue in substantially inactivated one-chain form capable of being converted to the active two-chain form, which analogue is modified in the region from position 414 to position 419 or from position 419 to position 426 or from position 426 to position 433.

2. A t-PA analogue according to claim 1, in which the interaction of a positively charged amino acid residue in position 419 and optionally also in position 420 with a negatively charged amino acid residue in position 480 is inhibited.

3. A t-PA analogue according to claim 1 or 2 which has no positively charged amino acid residue in position 419.

4. A t-PA analogue according to claim 3, wherein the positively charged amino acid residue in position 419 has been replaced by a negatively charged or uncharged amino acid residue.

5. A t-PA analogue according to claim 4, wherein Lys(419) of native t-PA has been replaced by an amino acid residue selected from the group consisting of Ser, Thr, Glu, Val, Ala, Leu, Ile, Tyr, Asn, Asp and Gln.

6. A t-PA analgue according to claim 2, which is further modified in position 420.

7. A t-PA analogue according to claim 6, wherein the positively charged amino acid residue in position 420 has been replaced by a negatively charged or uncharged amino acid residue.

8. A t-PA analogue according to claim 7, wherein His(420) of native t-PA has been replaced by an amino acid residue selected from the group consisting of Ser, Thr, Glu, Val, Ala, Leu, Ile, Tyr, Asn, Asp and Gln.

9. A t-PA analogue according to any of claims 1-8, which additionally comprises a modification in the A-chain.

10. A t-PA analogue according to claim 9, which additionally is modified in or lacks the growth factor sequence.

11. A t-PA analogue according to claim 9, which additionally is modified in the finger domain.

12. A t-PA analogue according to claim 9, which additionally lacks kringle 1.

13. A t-PA analogue according to claim 9, which additionally lacks the finger and growth factor sequences.

14. A t-PA analogue according to claim 9, wherein kringle 1 has additionally been replaced by kringle 1 of plasminogen.

15. A DNA construct which comprises a DNA sequence encoding a t-PA analogue which is modified in the region from position 414 to position 419 or from position 419 to position 426 or from position 426 to position 433.

16. A DNA construct according to claim 15, wherein a codon specifying a positively charged amino acid in position 419 of the t-PA analogue encoded by the DNA sequence has been replaced by another codon specifying a negatively charged or uncharged amino acid.

17. A DNA construct according to claim 16, wherein a codon specifying a positively charged amino acid in position 420 of the t-PA analogue encoded by the DNA sequence has additionally been replaced by another codon specifying a negatively charged or uncharged amino acid.

18. A DNA construct according to claim 16 or 17, which has a DNA sequence substantially as shown in Fig. 2 appended hereto, or a suitable modification thereof.

19. A recombinant expression vector which carries an inserted DNA construct according to any of claims 15-18.

20. A host cell which is transformed or transfected with the expression vector according to claim 19.

21. A pharmaceutical composition which comprises a t-PA analogue according to any of claims 1-14 and a pharmaceutically acceptable diluent or vehicle.

22. A t-pA analogue according to any of claims 1-14 for the treatment of diseases or disorders associated with the formation of thrombi in blood vessels.

23. Use of a t-PA analogue according to any of claims 1-14 for the preparation of a medicament for the treatment of diseases or disorders associated with the formation of thrombi in blood vessels.

24. A method of preparing a t-PA analogue in inactivated one-chain form, the method comprising

(a) modifying a DNA sequence encoding native t-PA by replacing a codon specifying a positively charged amino acid in position 419 of native t-PA by a negatively charged or uncharged amino acid,

(b) inserting the DNA sequence prepared in step (a) into a suitable replicable expression vector,

(c) transforming or transfecting a suitable host cell with the expression vector prepared in step (b), and

(d) growing the transformed host cell in a suitable growth medium under conditions conducive to the expression of the t-PA analogue, and recovering the analogue from the culture.

25. A method according to claim 24 wherein, in step (a) of the method, a codon specifying a positively charged amino acid in position 420 of native t-PA is replaced by a codon specifying a negatively charged or uncharged amino acid.

# Fig. 1A

Synthetic oligonucleotides I—VI

EP 0 351 246 A2

Fig. 1B

# Fig. 1C

# Fig. 2

mutation with NOR518

t-PA 3'end

SacI · LysHis SerThr · BamHI

pt-PA-937[K419S,H420T]

pt-PABam

BamHI/SacI

1.35kb fragment
t-PA 5'end

BamHI/SacI
0.43kb fragment
t-PA 3'end

Zem219b/BamHI

alkaline phosphatase

pt-PA[K419S,H420T]

PUC 18

Amp res.

EcoRI 6601 BamHI EcoRI NarI BglII

HindIII
ScaI
6000
SV40 DHFR cDNA · SV40 prom · MT · Human mutant tPA cDNA

NarI
EcoRI
1000
ScaI

poly(A) signal
EcoRI

EcoRI
LysHis to SerThr.
ScaI

5000

BamHI
2000
poly(A) signal

Human GH term.

EcoRi
HindIII
NarI

4000

ScaI

3000

# Fig. 3

mutation with NOR517

tPA 3'end

SacI      LysHis      BamHI
          SerThr

pt-PA-937[K419S]

BamHI/SacI
0.43kb fragment          Zem219b/BamHI
t-PA 3'end

pt-PABam                                          alkaline phosphatase

BamHI/SacI

1.35kb fragment
t-PA 5'end

6601
                                                BamHI
                    EcoRI                       EcoRI
                                                NarI
            HindIII                             BgIII
        ScaI                                              NarI
        6000                                                      EcoRI
                      SV40                                         1000
                      prom    MT                                  ScaI
    poly(A)                                                           EcoRI
    signal
    EcoRI                                                            Lys to Ser.

5000

                    pt-PA[K419S]

                                                                    BamHI
                                                                    2000
                    pUC 18                                          poly(A)
                                                                    signal
            Amp res.

                                                                    EcoRI
    4000                                                          HindIII
                                                              NarI
            ScaI              3000

SV40 DHFR cDNA    Human mutant tPA cDNA    Human GH term.

# Fig. 4

```
      BamHI EcoRI    NcoI
   1  GGATCCGAATTCCACCATGGATGCAATGAAGAGAGGGCTCTGCTGTGT    48
                     M  D  A  M  K  R  G  L  C  C  V
      |------>Synthetic 5'adaptor


                     NarI
  49  GCTGCTGCTGTGTGGCGCCGTCTTCGTTTCGCCCAGCCAGGAAATCCA    96
       L  L  L  C  G  A  V  F  V  S  P  S  Q  E  I  H


                              BglII
  97  TGCCCGATTCAGAAGAGGAGCCAGATCTTACCAAGTGATCTGCAGAGA    144
       A  R  F  R  R  G  A  R  S  Y  Q  V  I  C  R  D
      Synthetic 5' adaptor <------|


 145  TGAAAAAACGCAGATGATATACCAGCAACATCAGTCATGGCTGCGCCC    192
       E  K  T  Q  M  I  Y  Q  Q  H  Q  S  W  L  R  P


 193  TGTGCTCAGAAGCAACCGGGTGGAATATTGCTGGTGCAACAGTGGCAG    240
       V  L  R  S  N  R  V  E  Y  C  W  C  N  S  G  R


 241  GGCACAGTGCCACTCAGTGCCTGTCAAAAGTTGCAGCGAGCCAAGGTG    288
       A  Q  C  H  S  V  P  V  K  S  C  S  E  P  R  C


 289  TTTCAACGGGGGCACCTGCCAGCAGGCCCTGTACTTCTCAGATTTCGT    336
       F  N  G  G  T  C  Q  Q  A  L  Y  F  S  D  F  V


 337  GTGCCAGTGCCCCGAAGGATTTGCTGGGAAGTGCTGTGAAATAGATAC    384
       C  Q  C  P  E  G  F  A  G  K  C  C  E  I  D  T
                                              ClaI
                t-PA C87S,K419S:     AGT        C
                                      S


 385  CAGGGCCACGTGCTACGAGGACCAGGGCATCAGCTACAGGGGCACGTG    432
       R  A  T  C  Y  E  D  Q  G  I  S  Y  R  G  T  W


 433  GAGCACAGCGGAGAGTGGCGCCGAGTGCACCAACTGGAACAGCAGCGC    480
       S  T  A  E  S  G  A  E  C  T  N  W  N  S  S  A


 481  GTTGGCCCAGAAGCCCTACAGCGGGCGGAGGCCAGACGCCATCAGGCT    528
       L  A  Q  K  P  Y  S  G  R  R  P  D  A  I  R  L
```

# Fig. 5A

529 GGGCCTGGGGAACCACAACTACTGCAGAAACCCAGATAGAGACTCAAA 576
    G   L   G   N   H   N   Y   C   R   N   P   D   R   D   S   K

577 GCCCTGGTGCTACGTCTTTAAGGCGGGGAAGTACAGCTCAGAGTTCTG 624
    P   W   C   Y   V   F   K   A   G   K   Y   S   S   E   F   C

625 CAGCACCCCTGCCTGCTCTGAGGGAAACAGTGACTGCTACTTTGGGAA 672
    S   T   P   A   C   S   E   G   N   S   D   C   Y   F   G   N

673 TGGGTCAGCCTACCGTGGCACGCACAGCCTCACCGAGTCGGGTGCCTC 720
    G   S   A   Y   R   G   T   H   S   L   T   E   S   G   A   S

721 CTGCCTCCCGTGGAATTCCATGATCCTGATAGGCAAGGTTTACACAGC 768
    C   L   P   W   N   S   M   I   L   I   G   K   V   Y   T   A

769 ACAGAACCCCAGTGCCCAGGCACTGGGCCTGGGCAAACATAATTACTG 816
    Q   N   P   S   A   Q   A   L   G   L   G   K   H   N   Y   C

817 CCGGAATCCTGATGGGGATGCCAAGCCCTGGTGCCACGTGCTGAAGAA 864
    R   N   P   D   G   D   A   K   P   W   C   H   V   L   K   N

865 CCGCAGGCTGACGTGGGAGTACTGTGATGTGCCCTCCTGCTCCACCTG 912
    R   R   L   T   W   E   Y   C   D   V   P   S   C   S   T   C

913 CGGCCTGAGACAGTACAGCCAGCCTCAGTTTCGCATCAAAGGAGGGCT 960
    G   L   R   Q   Y   S   Q   P   Q   F   R   I   K   G   G   L

961 CTTCGCCGACATCGCCTCCCACCCCTGGCAGGCTGCCATCTTTGCCAA 1008
    F   A   D   I   A   S   H   P   W   Q   A   A   I   F   A   K

1009 GCACAGGAGGTCGCCCGGAGAGCGGTTCCTGTGCGGGGGCATACTCAT 1056
    H   R   R   S   P   G   E   R   F   L   C   G   G   I   L   I

# Fig. 5B

```
1057  CAGCTCCTGCTGGATTCTCTCTGCCGCCCACTGCTTCCAGGAGAGGTT    1104
        S   S   C   W   I   L   S   A   A   H   C   F   Q   E   R   F


1105  TCCGCCCCACCACCTGACGGTGATCTTGGGCAGAACATACCGGGTGGT    1152
        P   P   H   H   L   T   V   I   L   G   R   T   Y   R   V   V


1153  CCCTGGCGAGGAGGAGCAGAAATTTGAAGTCGAAAAATACATTGTCCA    1200
        P   G   E   E   E   Q   K   F   E   V   E   K   Y   I   V   H


1201  TAAGGAATTCGATGATGACACTTACGACAATGACATTGCGCTGCTGCA    1248
        K   E   F   D   D   D   T   Y   D   N   D   I   A   L   L   Q


1249  GCTGAAATCGGATTCGTCCCGCTGTGCCCAGGAGAGCAGCGTGGTCCG    1296
        L   K   S   D   S   S   R   C   A   Q   E   S   S   V   V   R


1297  CACTGTGTGCCTTCCCCCGGCGGACCTGCAGCTGCCGGACTGGACGGA    1344
        T   V   C   L   P   P   A   D   L   Q   L   P   D   W   T   E
```

```
                    SacI
1345  GTGTGAGCTCTCCGGCTACGGCAAGCATGAGGCCTTGTCTCCTTTCTA    1392
        C   E   L   S   G   Y   G   K   H   E   A   L   S   P   F   Y
                                        ScaI
      t-PA K419S,H420T:                 AGTACT
                                        S   T
      t-PA K419S:                       TCC
                                        S
      t-PA C87S,K419S:                  TCC
                                        S
```

```
1393  TTCGGAGCGGCTGAAGGAGGCTCATGTCAGACTGTACCCATCCAGCCG    1440
        S   E   R   L   K   E   A   H   V   R   L   Y   P   S   S   R


1441  CTGCACATCACAACATTTACTTAACAGAACAGTCACCGACAACATGCT    1488
        C   T   S   Q   H   L   L   N   R   T   V   T   D   N   M   L


1489  GTGTGCTGGAGACACTCGGAGCGGCGGGCCCCAGGCAAACTTGCACGA    1536
        C   A   G   D   T   R   S   G   G   P   Q   A   N   L   H   D


1537  CGCCTGCCAGGGCGATTCGGGAGGCCCCCTGGTGTGTCTGAACGATGG    1584
        A   C   Q   G   D   S   G   G   P   L   V   C   L   N   D   G
```

# Fig. 5C

1585  CCGCATGACTTTGGTGGGCATCATCAGCTGGGGCCTGGGCTGTGGACA  1632
      R   M   T   L   V   G   I   I   S   W   G   L   G   C   G   Q


1633  GAAGGATGTCCCGGGTGTGTACACAAAGGTTACCAACTACCTAGACTG  1680
      K   D   V   P   G   V   Y   T   K   V   T   N   Y   L   D   W


1681  GATTCGTGACAACATGCGACCGTGACCAGGAACACCCGACTCCTCAAA  1728
      I   R   D   N   M   R   P  End


                                    SalI   PstI           BamHI
1729  AGCAAATGAGATCCGATAAGCTCTAGTCGACCTGCAGCCCAAGCTCGG  1776
                  |———>            Synthetic 3' adaptor


1777  ATCC   1780
       <———|


# Fig. 5D

# Fig. 6A

# Fig. 6B

## Fig. 7

Fig. 8

Rabbit thrombolysis model
Dissolved $^{125}$I—fibrin after 1 h

% Thrombolysis

t—PA dosage (ug/kg/h)

EP 0 351 246 A2